# NEW EUROPEAN PATENT SPECIFICATION

(11) **EP 1 438 961 B2**
(45) Date of publication and mention of the opposition decision: **20.08.2014**
(45) Mention of the grant of the patent: 21.03.2007
(21) Application number: 03290092.0
(22) Date of filing: 14.01.2003
(51) Int. Cl.: A61K 31/496, A61K 9/16, A61K 9/20

(54) **Bioequivalent composition of itraconazole dispersed in a hydrophilic polymer**
Bioaequivalente Zusammensetzung in der Form einer festen Dispersion enthaltend Itraconazol und ein hydrophiles Polymer
Composition bioéquivalente sous la forme d'une dispersion solide comprenant de l'itraconazole et un polymer hydrophile

(43) Date of publication of application: 21.07.2004
(73) Proprietor: Acino Pharma AG, 4253 Liesberg (CH)
(72) Inventor: Seth, Pawan, Irvine, CA 92612 (US)
(74) Representative: Keller, Günter

(56) References cited:
- EP-A- 1 103 252
- WO-A-98/42318
- WO-A1-00/03697
- WO-A1-01/41765
- WO-A1-94/05263
- WO-A1-98/31360
- US-A- 5 633 015
- US-A1- 2001 007 678
- US-A1- 2002 176 894
- US-B1- 6 485 743
- CHOWDARY K P R ET AL: "DISSOLUTION RATE AND FORMULATION STUDIES ON SOLID DISPERSIONS OF ITRACONAZOLE" INDIAN JOURNAL OF PHARMACEUTICAL SCIENCES, XX, XX, vol. 62, no. 6, November 2000 (2000-11), pages 471-474, XP001105434 ISSN: 0250-474X

## Description

### BACKROUND OF THE INVENTION

The invention relates to an itraconazole composition having a bioequivalency dissolution, and to a process for preparing it.

Low-solubility pharmaceutical compounds often exhibit the drawback of insufficient dissolution in gastric fluids that prejudices obtaining a plasmatic concentration sufficient to achieve the therapeutical effects. Much work has been undertaken to achieve a sufficient plasmatic concentration necessary to reach the desired therapeutic effects.

Addition of a surfactant is a simple way to enhance dissolution. It allows to raise wettability of otherwise poorly-wettable drugs and hence to increase the dissolution rate of the drug. This technique, rather old, has been improved over the last years.

EP-A-0330532 provides co-micronization of a poorly-soluble drug (fenofibrate) with a surfactant (sodium laurylsulfate). The result is better than that obtained with pure mixing of the components.

WO-A-9831360 discloses fluidized bed granulation on an inert support of a suspension of a drug, where the suspension is in an aqueous solution comprising the hydrophilic polymer. Itraconazole is mentioned as a possible drug to which this invention may apply.

EP-A-1 103 252 discloses a pharmaceutical oral preparation of itraconazole comprising itraconazol (19-30 wt%), a hydrophilic polymer (25-60 wt%), a surfactant (1-15 wt%, preferably 3-10 wt%) and an inert core (10-45 wt%).

US-A-2002/0176894 discloses a pharmaceutical composition comprising itraconazole and as preferred emulsifier vitamin E, Polyethylene Glycol Succinate.

There is a need for an itraconazole that would be bioequivalent, when compared to the existing marketed compositions such as Sporanox^{®}, and which would be easier to manufacture, compared to the existing compositions. None of the above documents teaches or suggests the instant invention.

### SUMMARY OF THE INVENTION

The invention provides an itraconazole composition comprising itraconazole dispersed in a hydrophilic polymer, hydroxypropylmethylcellulose and a surfactant namely(polyoxyethylene) sorbitane monostearate having a dissolution at 30 minutes between 10 and 40%, at 45 minutes between 25 and 80%, at 60 minutes between 60 and 95%, at 90 minutes at least 80%, as measured using the type II paddle method at 100 rpm according to the US Pharmacopoeia, in a dissolution medium comprised of 1000 ml HCl 0.1N, wherein the surfactant represents between 0.1 and 3 wt%.

The weight ratio polymer:itraconazole is lower than 1.9.

The composition comprises an inert carrier covered with at least one layer containing said itraconazole dispersed in a hydrophilic polymer and a surfactant, wherein the weight ratio polymer:itraconazole is lower than 1.9.

Said ratio may be from 0.5 to 1.9, especially from 1 to 1.9.

The invention also provides a process for preparing a composition of the invention comprising the steps of:
(i) preparing a solution of itraconazole and polymer, and a surfactant, in an organic solvent, wherein the weight ratio polymer:itraconazole is lower than 1.9; and
(ii) applying said solution onto an inert carrier.

### DETAILED DESCRIPTION

The invention is designed for itraconazole. The composition of the invention comprises the ingredients according to the following proportions: itraconazole: between 6 and 30wt%; polymer: between 10 and 60wt%; surfactant: between 0.1 and 3wt% and inert carrier: between 20 and 80wt%.

The inventor has found that surprisingly, when the granule as defined below is placed in an aqueous medium, dissolution provides bioequivalency, compared to existing formulations, in the case the weight ratio polymer:itraconazole is lower than 1.9 (preferably equal to or lower than 1.85, more preferably equal to or lower than 1.8).

Also, the inventor has found that the invention provides surprising results, when compared to prior art techniques, i.e. fluidized bed granulation with a suspension.

In the framework of this invention, the expression "inert carrier" means any excipient, generally hydrophilic, pharmaceutically inert, crystalline or amorphous, in a particulate form, not leading to a chemical reaction under the operating conditions employed, and which is preferably soluble in an aqueous medium, notably in a gastric acid medium. Examples of such excipients are derivatives of sugars, such as lactose, saccharose, hydrolyzed starch (malto-dextrine), saccharose, mannitol, microcrystalline cellulose, etc. Preferably inert spheroids are used, such as those disclosed in "sugar spheres, US Pharmacopea XXV". These inert spheroids may be obtained by many processes, including extrusion of a water paste, followed by spheronization. These are available from Seppic, France or Werner, Germany. Mixtures are also suitable. The individual particle size of the inert hydrosoluble carrier can be, for example, between 50 and 500 microns, e.g. between 100 and 400 microns.

The expression "hydrophilic polymer" in the invention should be taken to mean hydroxypropylmethylcellulose.

The hydrophylic polymer is HPMC. The HPMC used in this invention is, for example, such that a 2% aqueous solution of the polymer at 20°C has a viscosity between 0,5 and 50cP, preferably between 2 and 25cP.

The surfactant is the monostearate ester of (polyoxyethylene) sorbitane.

Additional agent, known in the art can be used together with the drug and the polymer, albeit this is not preferred.

The composition according to the invention is prepared by a novel process comprising spraying a solution of itraconazole and the hydrophilic polymer and the surfactant, onto the inert cores.

The method according to the invention consists in using the fluidized bed granulation principle, but with specific starting materials, in order to arrive at an improved dissolution profile and thus, at elevated bioavailability. In particular, the invention employs a solution of itraconazole in a solution of the hydrophylic polymer and the surfactant, where the solvent is an organic solvent. The solvent will solubilize both itraconazole and the polymer. This solvent can be a chlorinated solvent, such as methylene chloride (aka dichloromethane), or an alcane, such as hexane. The chlorinated solvent is preferred. It can be mixed with a co-solvent such as an alcohol, e.g. ethyl alcohol or isopropyl alcohol. The amount of the co-solvent can be up to 50% by volume, preferably up to 40%, especially between 25 and 40%.

The fluidized-bed granulation technique is widely used in the pharmaceutical industry for preparing capsules or tablets. Conventionally, according to the prior art, a powder or a mixture of powders (itraconazole + excipients) is put into suspension in the fluidized bed in a granulator, and a solution containing a binder and, optionally, a surfactant, is sprayed onto this bed to form granules. The fluidized-bed granulation technique is well known to those skilled in the art and reference should be made to standard works such as for example "Die Tablette", by Ritschel, Ed. Cantor Aulendorf, pages 211-212.

The invention, as has been indicated, comprises spraying a solution in an organic solvent of itraconazole with the hydrophilic polymer and the surfactant onto an inert carrier. Following granulation, the granule formed consists of crystals of, for example, lactose, which are isolated (or possibly agglomerated together by the spray solution) and particles of itraconazole and polymer adhering to the crystal surface. The granule could similarly be constituted of coated crystals, which are agglomerated, or even of such an agglomerate having received a coating. The granule obtained with the instant process can be broadly defined as comprising inert carrier particles, which are either isolated or agglomerated together, and particles of itraconazole in admixture with the hydrophilic polymer, adhering to the carrier particles surface.

The significant starting product is the solution of itraconazole. This solution is prepared by putting the itraconazole into solution in a solution comprising the hydrophylic polymer and the surfactant, in solution in an organic solvent. The surfactant is put into solution in the solvent (beaker + magnetic or vane stirrer). Next, the hydrophylic polymer (HPMC) is dispersed, while stirring, in the solution previously obtained. While still stirring, the itraconazole is dispersed in the form of a fine shower into the above solution, to form a solution. The order of these steps can be reversed. In case a co-solvent is used, part of the components can first be dissolved (or otherwise solubilized) in this co-solvent prior to its admixing with the solvent.

The itraconazole concentration in the solution is generally from 1 to 15% by weight, preferably from 2 to 10%. The hydrophylic polymer concentration in the solution is generally from 1 to 25% by weight, preferably 3 to 20%. The surfactant concentration in the solution is from 0.1 to 3% by weight, preferably below 2%. The total components (incl. but not limited to the three above) content in the solution is from 3 to 20% by weight, preferably 5 to 15%.

Further studies have shown that is possible to specifically adapt the amount of solvent used, so as to avoid at the same time sticking problems due to too high concentration (more than 20%) and excessive spray rate due to too large volumes (more than 20g/minute/kg of carrier particles).

The granules thus obtained can, if desired, be provided with an outer coating or compressed into tablets, or form agglomerates. The outer coating is applied using conventional coating techniques such as coating in a pan or fluidized bed coater.

The granule may be available for the patient in many forms, including in a hard capsule or in a tablet form. When administered in a capsule form, the granules will generally be mixed with a lubricant. When administered in a tablet form, the granules will generally be mixed with a disintegrant and a lubricant. The granules will generally represent 50 to 90% of the final weight of the pharmaceutical composition (tablet or capsule).

Examples of disintegrants are cross-linked polyvinylpyrrolidone, sodium croscarmellose, sodium carboxymethyl starch, modified starch or unmodified starch. Examples of lubricants are magnesium stearate, sodium stearyl fumarate, glycerol behenate and talc. Flow enhancing agents (such as colloidal silica) may also be used. The excipients can be any one traditionally used in the art. For more details about these excipients, one can refer to the disclosure in "Handbook of pharmaceutical excipients", American Pharmaceutical Association, 1994 ISBN 0 91730 66 8, by Wade A. and Weller P. When the granule obtained (whether subsequently coated or not) is compressed to form tablets, this step can be implemented using any conventional technique which is suitable, for example using alternating or rotating compressing equipment.

### EXAMPLES

The following examples illustrate the invention without limiting it.

### Example 1.

The following formulations are prepared, where Ex. 1A is the prior art technique (suspension) while Ex. 1B is in accordance with the invention.

| Compound | Amount (g) | |
|---|---|---|
| | Ex. 1A | Ex. 1B |
| Itraconazole | 100 | 100 |
| HPMC | 160 | 180 |
| Sorbitan monostearate | 0 | 5 |
| Sodium lauryl sulfate | 5 | 0 |
| Sugar spheres 20-25 mesh | 196.75 | 195 |
| Methylene chloride | - | 1647 |
| Ethyl alcohol | - | 910 |
| Water | 937 | - |

Surfactant is dissolved in water (Ex. 1A) or alcohol (Ex. 1B). Itraconazole is suspended in water (Ex. 1A) or dissolved in methylene chloride (Ex. 1B). HPMC is dissolved in water (Ex. 1A) or alcohol (Ex. 1B). Suspension (Ex. 1A) or solution (Ex. 1B) is sprayed on the sugar spheres in a fluidized bed granulator (Glatt^{®} GPCG1), equipped with a bottom injection system (Wurster^{®}). The following parameters are used.

| | |
|---|---|
| Inlet Temperature (°C) | 45-48°C |
| Product Temperature (°C) | 40-45°C |
| Air Volume (m³/h) | 100-130 |
| Atomization Pressure (bar) | 2.5-3.0 |
| Nozzle diameter (mm) | 1.0 |
| Pump Speed (g/min) | 8-13 |

Dissolution tests are carried out, in an apparatus according to the US Pharmacopea, XXIV, type II paddle, at 100 rpm, in a dissolution medium comprised of 1000 ml of HC1 0.1N.

The results (expressed in dissolved fraction in %) are the following.

| Time (min) 0 | | 15 | 30 | 45 | 60 | 90 |
|---|---|---|---|---|---|---|
| 1A | 0 | 10 | 15 | 15 | 15 | 15 |
| 1B | 0 | 5 | 29 | 72 | 87 | 91 |

Results clearly show that the release in the case of the invention is far different from the prior art.

### Example 2.

The following formulation is prepared.

| Compound | Amount (g) |
|---|---|
| Itraconazole | 100 |
| HPMC | 185 |
| Sorbitan monostearate | 5 |
| Sugar spheres 20-25 mesh | 195 |
| Methylene chloride | 1650 |
| Ethyl alcohol | 900 |

Surfactant is dissolved in alcohol under moderate stirring. HPMC then itraconazole are added to alcohol. Methylene chloride is then gradually added, resulting in solubilizing itraconazole and HPMC. The resulting solution is filtered on a mesh (100 mesh). It is then sprayed on the sugar spheres in fluidized bed granulator (Glatt^{®} GPCG1), equipped with a top injection system (Top Spray^{®}). The following parameters are used.

| | |
|---|---|
| Inlet Temperature(°C) | 45-48°C |
| Product Temperature (°C) | 40-45°C |
| Air Volume (m³/h) | 100-130 |
| Atomization Pressure (bar) | 2.5-3.0 |
| Nozzle diameter (mm) | 1.0 |
| Pump Speed (g/min) | 8-13 |

The resulting granules are filled in hard capsules (together with 15g of silica as filler). These capsules are then subjected to a dissolution test identical to example 1.

The results (expressed in dissolved fraction in %) are the following.

| Time (min) | 0 | 15 | 30 | 45 | 60 | 90 |
|---|---|---|---|---|---|---|
| | 0 | 5 | 17 | 39 | 74 | 97 |

Results clearly show that the release in the case of the invention makes it bioequivalent to the existing Sporanox^{®} product.

## Claims

1. An itraconazole composition comprising an inert carrier covered with at least one layer containing said itraconazole dispersed in a hydroxypropylmethylcellulose and (polyoxyethylene) sorbitane monostearate, wherein the weight ratio polymer:itraconazole is lower than 1.9,
wherein the surfactant represents between 0.1 and 3 wt%,
the itraconazole represents between 6 and 30 wt%,
the polymer represents between 10 and 60 wt%, and
the inert carrier represents between 20 and 80 wt%,
having a dissolution at 30 minutes between 10 and 40%, at 45 minutes between 25 and 80%, at 60 minutes between 60 and 95%, at 90 minutes at least 80%, as measured using the type II paddle method at 100 rpm according to the US Pharmacopoeia, in a dissolution medium comprised of 1000 ml HCl 0.1N.

2. The composition of claim 1, wherein the inert carrier is an inert sugar sphere.

3. The composition of any one of claims 1 to 2, wherein the inert carrier has a particle size between 50 and 500 microns.

4. The composition of any one of claims 1 to 3 in a capsule.

5. The composition of any one of claims 1 to 3 compressed into a tablet.

6. A process for preparing a composition of any one of claims 1 to 5 comprising the steps of:
(i) preparing a solution of itraconazole and polymer, and a surfactant in an organic solvent, wherein the weight ratio polymer:itraconazole is lower than 1.9; and
(ii) applying said solution onto an inert carrier.

7. The process of claim 6, wherein step (ii) comprises fluidized bed granulating.

8. The process of claim 6 or 7 wherein in step (i) the solvent is a chlorinated solvent, optionally in admixture with an alcohol.

9. The process of claim 8, wherein the solvent is methylene chloride, optionally in admixture with an alcohol.

## Patentansprüche

1. Itraconazolzusammensetzung, die einen inerten Träger umfasst, der mit mindestens einer Schicht bedeckt ist, die das in Hydroxypropylmethylcellulose und (Polyoxyethylen-) Sorbitanmonostearat dispergierte Itraconazol enthält, wobei das Gewichtsverhältnis Polymer:Itraconazol weniger als 1,9 beträgt,
wobei das Tensid zwischen 0,1 und 3 Gew.% darstellt,
das Itraconazol zwischen 6 und 30 Gew.% darstellt,
das Polymer zwischen 10 und 60 Gew.% darstellt,
der inerte Träger zwischen 20 und 80 Gew.% darstellt,
mit einer Löslichkeit bei 30 Minuten zwischen 10 und 40%, bei 45 Minuten zwischen 25 und 80%, bei 60 Minuten zwischen 60 und 95%, bei 90 Minuten von mindestens 80%, gemessen unter Verwendung der Paddle-Methode des Typs II bei 100 UpM gemäß der US Pharmakopöe, in einem Lösungsmedium, das 1000 ml 0,1 N HCl umfasst.

2. Zusammensetzung nach Anspruch 1, wobei der inerte Träger ein inertes Zuckerkügelchen ist.

3. Zusammensetzung nach einem der Ansprüche 1 bis 2, wobei der inerte Träger eine Teilchengröße zwischen 50 und 500 Mikron aufweist.

4. Zusammensetzung nach einem der Ansprüche 1 bis 3 in einer Kapsel.

5. Zusammensetzung nach einem der Ansprüche 1 bis 3 zu einer Tablette komprimiert.

6. Verfahren zur Herstellung einer Zusammensetzung nach einem der Ansprüche 1 bis 5, welches die Schritte umfasst:
(i) Herstellen einer Lösung von Itraconazol und Polymer sowie eines Tensids in einem organischen Lösungsmittel, wobei das Gewichtsverhältnis Polymer:Itraconazol weniger als 1,9 beträgt; und
(ii) Auftragen dieser Lösung auf einen inerten Träger.

7. Das Verfahren nach Anspruch 6, wobei der Schritt (ii) Wirbelschicht-Sprühgranulation umfasst.

8. Verfahren nach Anspruch 6 oder 7, wobei in Schritt (i) das Lösemittel ein chloriertes Lösemittel, wahlweise unter Beimischung eines Alkohols ist.

9. Verfahren nach Anspruch 8, wobei das Lösemittel Methylenchlorid, wahlweise unter Beimischung eines Alkohols ist.

## Revendications

1. Une composition d'itraconazole comprenant un support inerte recouvert d'au moins une couche contenant ledit itraconazole dispersé dans un hydroxypropylméthylcellulose et un (polyoxyéthylène) monostéarate de sorbitan, dans laquelle le rapport pondéral polymère:itraconazole est inférieur à 1,9,
dans laquelle l'agent tensioactif représente 0,1 à 3% en poids de la composition, l'itraconazole représente 6 à 30% en poids de la composition,
le polymère représente 10 à 60% en poids de la composition, et
le support inerte représente 20 à 80% en poids de la composition,
la composition présentant un taux de dissolution à 30 minutes compris entre 10 et 40%, à 45 minutes compris entre 25 et 80%, à 60 minutes compris entre 60 et 95%, à 90 minutes d'au moins 80%, déterminé par la méthode de l'aube de type II à 100 tpm telle que définie par la Pharmacopée US, dans un milieu de dissolution comprenant 1000 ml de HCL 0,1 N.

2. La composition selon la revendication 1, dans laquelle le support inerte est une sphère de sucre inerte.

3. La composition selon une quelconque des revendications 1 à 2, dans laquelle le support inerte présente une dimension particulaire comprise entre 50 et 500 microns.

4. La composition selon une quelconque des revendications 1 à 3, sous forme de capsule.

5. La composition selon une quelconque des revendications 1 à 3, compressé sous forme de comprimé.

6. Un procédé de préparation d'une composition selon une quelconque des revendications 1 à 5, comprenant les étapes de:
(i) préparation d'une solution d'itraconazole et d'un polymère, et d'un agent tensioactif dans un solvant organique, dans laquelle le rapport pondéral polymère:itraconazole est inférieur à 1,9; et
(ii) application de ladite solution sur un support inerte.

7. Le procédé selon la revendication 6, dans lequel l'étape (ii) comprend une granulation par lit fluidisé.

8. Le procédé selon la revendication 6 ou 7, dans lequel dans l'étape (i) le solvant est un solvant chloré, éventuellement mélangé à un alcool.

9. Le procédé selon la revendication 8, dans lequel [Anmerkung s. Anspruch 8] le solvant est le chlorure de méthyle, éventuellement mélangé à un alcool.
